# EUROPÄISCHE PATENTANMELDUNG

(11) **EP 0 643 950 A1**
(43) Veröffentlichungstag der Anmeldung: **22.03.1995**
(21) Anmeldenummer: 94110817.7
(22) Anmeldetag: 12.07.1994
(51) Int. Cl.: A61C 19/00

(54) **Lichtzuführeinrichtung**

(30) Priorität: 20.09.1993 DE 4331859
(71) Anmelder: HERAEUS KULZER GMBH, D-61273 Wehrheim (DE)
(72) Erfinder: Schödel, Dieter Dr., 65193 Wiesbaden (DE); Oppawsky, Steffen, D-61350 Bad Homburg (DE); Grigereit, Helmut, Laguna Miquel, Cal-92677 (US)
(74) Vertreter: Grimm, Ekkehard, Dipl.-Phys.

(57) **Zusammenfassung**

Es ist eine Lichtzuführeinrichtung für medizinische und/oder zahntechnische Anwendungen mit einem Lichtleiter und einem Lichtaustrittsteil an deren einem Ende, das in einem Griffstück gehalten ist, bekannt, wobei das Griffstück (1) lösbar mittels Rastelementen mit einer Verbindungshülse (5) verbunden ist, die ihrerseits unverrückbar an dem Lichtleiter befestigt ist, wobei die Rastelemente durch Vorsprünge und diesen zugeordneten Vertiefungen gebildet sind. Um eine Lichtzuführeinrichtung für medizinische und/oder zahntechnische Anwendungen anzugeben, die auf einfache Weise die Möglichkeit besitzt, die kontaminierten Teile nach einer Behandlung eines Patienten zu reinigen bzw. auszutauschen, sind die Vorsprünge und die Vertiefungen Teile einer Drehverbindung, wobei die Vorsprünge als Stege (8) ausgebildet sind.

## Beschreibung

Die Erfindung betrifft eine Lichtzuführeinrichtung für medizinische und/oder zahntechnische Anwendungen mit einem Lichtleiter und einem Lichtaustrittsteil an deren einem Ende, das in einem Griffstück gehalten ist, wobei das Griffstück lösbar mittels Rastelementen mit einer Verbindungshülse verbunden ist, die ihrerseits unverrückbar an dem Lichtleiter befestigt ist, wobei die Rastelemente durch Vorsprünge und diesen zugeordneten Vertiefungen gebildet sind.

Solche Lichtzuführeinrichtungen sind allgemein bekannt. Ein Lichtleiter der gattungsgemäßen Art ist darüberhinaus in der DE-OS 30 09 171 beschrieben. Diese Lichtzuführeinrichtung weist ein flexibles Mittelstück in Form eines Lichtleiters auf, an dessen einem Ende ein Verbindungsteil angeordnet ist, das mit einer Lichtquelle verbindbar ist. An dem anderen Ende ist ein Griffstück mit einem Lichtaustrittsteil vorgesehen. Derartige Lichtzuführeinrichtungen werden insbesondere im Zahnarztbereich eingesetzt, um z.B. polymerisierende Kunststoffe im Mund des Patienten auszuhärten.

Eine weitere Lichtzuführeinrichtung der eingangs beschriebenen Art mit einer stationären Lichtversorgung und einem flexiblen Lichtleiter, an dessen austrittsseitigem Ende ein Griffteil mit einem abgewinkelten Lichtaustrittsteil angeordnet ist, ist aus dem Firmenprospekt der Kulzer GmbH "Translux-Hochleistungs-Handlichtgeräte für Praxis und Labor" (151188/D 185 SK dt.) bekannt. Bei diesem Gerät stellt der flexible Lichtleiter mit dem Griffstück und dem Lichtaustrittsteil eine einzige Baueinheit dar, d.h. die werksseitig zusammenmontierten Teile können von dem Benutzer nicht in ihre einzelnen Bestandteile zerlegt werden.

Eine zunehmende Anforderung an solche Geräte besteht darin, insbesondere die Teile, die im Bereich des Mundes des Patienten zum Einsatz gelangen, zu reinigen bzw. zu sterilisieren.

Ausgehend von dem vorstehend beschriebenen Stand der Technik liegt der vorliegenden Erfindung die Aufgabe zugrunde, eine Lichtzuführeinrichtung für medizinische und/oder zahntechnische Anwendungen anzugeben, die auf einfache Weise die Möglichkeit besitzt, die kontaminierten Teile nach einer Behandlung eines Patienten zu reinigen bzw. auszutauschen.

Diese Aufgabe wird bei einer Lichtzuführeinrichtung der gattungsgemäßen Art dadurch gelöst, daß die Vorsprünge und die Vertiefungen Teile einer Drehverbindung sind, wobei die Vorsprünge als Stege ausgebildet sind. Durch diese Ausbildung kann das Griffstück mit dem darin befestigten Lichtaustrittsteil, beispielsweise einem festen Glasfaserstab, von dem flexiblen Lichtleiter abgenommen werden. Durch die Rastelemente in Form von Vorsprüngen und Vertiefungen an dem Griffstück einerseits und an der Verbindungshülse, die dem Lichtleiter zugeordnet ist, andererseits ist ein schneller Austausch des Griffstücks nach jeder Patientenbehandlung möglich. Die Drehverbindung bildet hierbei einen Schnellverschluß, der einerseits die miteinander verbundenen Bauteile sicher hält, andererseits eine exakte Zentrierung ermöglicht, die für die verlustfreie Einkopplung der Strahlung von dem flexiblen Lichtleiter in den Lichtleitstab des Griffstücks erforderlich ist.

Vorzugsweise werden zwei Stege vorgesehen, die bevorzugt gegenüberliegend angeordnet sind, d.h. die einlaufseitigen Enden der zwei Stege liegen diametral gegenüber. Hierdurch wird ein einfaches Verbinden des Griffstücks mit der Verbindungshülse ermöglicht. Die beiden zu verbindenden Teile, d.h. das Griffstück und die Verbinundgshülse werden aufeinandergesetzt und durch die Enden der Stege zentriert. Durch anschließendes leichtes Verdrehen der beiden Teile gegeneinander entgegen der Drehrichtung, in der sich die Teile verbinden, werden diese zusätzlich ausgerichtet und gleiten dann bei Drehung in Drehrichtung in die Vertiefungen ein.

Durch in Eindrehrichtung spitz zulaufende Stege wird eine leichte und exakte Einführung der Stege in die entsprechenden Vertiefungen des anderen Teils gewährleistet, wobei bei einer ausreichend breiten Ausbildung der Vorsprünge bzw. der diesen Vorsprüngen zugeordneten Vertiefungen oder Nuten ein einfaches Einführen der Stege in die Nuten gegeben ist.

Die Stege sind vorzugsweise an dem Außenumfang der Verbindungshülse angeordnet, so daß die Verbindungshülse auf einfache Weise als Spritzgußteil herstellbar ist. Darüberhinaus sind die Bereiche der Stege sehr gut für Reinigungszwecke zugänglich. Die dazu entsprechend ausgebildeten, gewindeförmigen Vertiefungen befinden sich auf dem Innenumfang des hülsenförmigen Griffstücks, die dort eingeschnitten und sehr einfach herstellbar sind.

Um sowohl ein leichtes Eindrehen der Verbindungshülse in das Griffstück zu gewährleisten als auch ein leichtes Lösen zum Austauschen des Griffstücks sicherzustellen, sind die Stege, die das Schraubgewinde bilden, an beiden Enden spitz zulaufend ausgebildet.

In einer weiteren als bevorzugt anzusehenden Ausbildungsform der Lichtzuführeinrichtung verlaufen die Stege derart, daß sie von dem einen spitzen Ende ausgehend in ihrer Breite zunehmen und von der breitesten Stelle unmittelbar wieder in eine Spitze auslaufen. Hierbei bildet in der einen Drehrichtung die eine Seite der einen Spitze eine kraftschlüssige Anlagefläche in den Vertiefungen, während in der anderen Drehrichtung die gegenüberliegende Seite der anderen Spitze die entsprechende kraftschlüssige Anlagefläche in den Vertiefungen bildet.

Die Vertiefungen können in Form von zwei parallel zueinander verlaufenden Gewindegängen am Innenumfang des Griffstücks gebildet sein, in die dann jeweils Stege der Verbindungshülse eingreifen. Durch diese zwei Gewindegänge werden zwei Einlauföffnungen gebildet, in die die Stege beim Verbinden des Griffstücks mit der Verbindungshülse gleichzeitig eingeführt und an zwei Stellen fixiert und verschraubt werden. Damit ist praktisch ein Verkanten der beiden Teile und damit ein Beschädigen des Gewindes ausgeschlossen.

Entsprechend sind in der einen bevorzugten Ausführungsform, insbesondere in Verbindung mit den zwei parallel zueinander verlaufenden Gewindegängen, am Innenumfang des Griffstücks zwei Stege vorgesehen, die, in Umfangsrichtung der Verbindungshülse bzw. des Griffstücks gesehen, gegenüberliegend verteilt sind. In einer vorteilhaften Dimensionierung ist die Steigung der Gewindegänge der Drehverbindung so gewählt, daß sich die Verbindungshülse und das Griffstück bei einer vollständigen Verdrehung gegeneinander um 360° um etwa 5 bis 10 mm, bevorzugt um etwa 10 mm, gegeneinander axial verschieben, bis sie fest miteinander gegen entsprechende Anschlagflächen anliegend ausgerichtet sind. Die Stege haben eine bevorzugte Breite von 1 bis 3 mm, wobei aus diesem Bereich einer Breite von 2 mm der Vorzug zu geben ist.

Damit das Griffstück mit dem Lichtaustrittsteil für eine Wiederverwendung leicht gereinigt werden kann, sollte zumindest das Griffstück aus sterilisierbarem Material hergestellt sein, das dann zu Reinigungszwecken in einem Sterilisator behandelt werden kann. Bevorzugt wird Polyehterimid verwendet, das chemikalienbeständig ist, eine hohe Wärmeformbeständigkeit aufweist und im Mund des Patienten unbedenklich ist.

Weitere Vorteile und Merkmale der Erfindung ergeben sich aus der nachfolgenden Beschreibung eines Ausführungsbeispiels anhand der Zeichnung. In der Zeichnung zeigt:
- Figur 1: einen Schnitt durch das Griffstück mit einem darin angekoppelten Lichtleiter entlang der Achse des Lichtleiters bzw. des Lichtaustrittsteils,
- Figur 2: das Griffstück der Lichtzuführungseinrichtung nach Figur 1 mit einem Teil des Lichtaustrittsteils,
- Figur 3: die Verbindungshülse der Lichtzuführeinrichtung nach Figur 1,
- Figur 4: eine vergrößerte Ausschnittsdarstellung der Verbindungshülse nach Figur 3 im Bereich der Stege der Drehverbindung, und
- Figur 5: einen Schnitt entlang der Schnittlinie V-V in Figur 4.

Die Lichtzuführeinrichtung weist ein Griffstück 1 auf, an dessen freiem Ende ein Lichtaustrittsteil in Form eines Lichtleiterstabs 2 eingeklebt ist. Der Lichtleiterstab 2 ist an seinem Ende gegenüber der Achse 3 der Lichtzuführeinrichtung in einem Winkel von etwa 120°, wie dies gezeigt ist, abgewinkelt, so daß dessen Lichtaustrittsfläche 4 beim Halten des Griffstücks 1 entsprechend einem Schreibgerät Strahlung nach schräg unten abgibt. Das Griffstück ist mit einer Verbindungshülse 5 lösbar verbunden, die an ihrem freien, aus dem Griffstück 1 vorstehenden Ende mit einem flexiblen Lichtleiter 6 verbunden, der zu einer nicht dargestellten Versorgungseinheit mit der Lichtquelle, beispielsweise einer Halogenlampe, führt.

Die lösbare Verbindung zwischen dem Griffstück 1 und der Verbindungshülse 5 wird durch eine Drehverbindung gebildet, wobei das Griffstück 1 Vertiefungen oder Nuten 7 und die Verbindungshülse 5 Stege 8 haben.

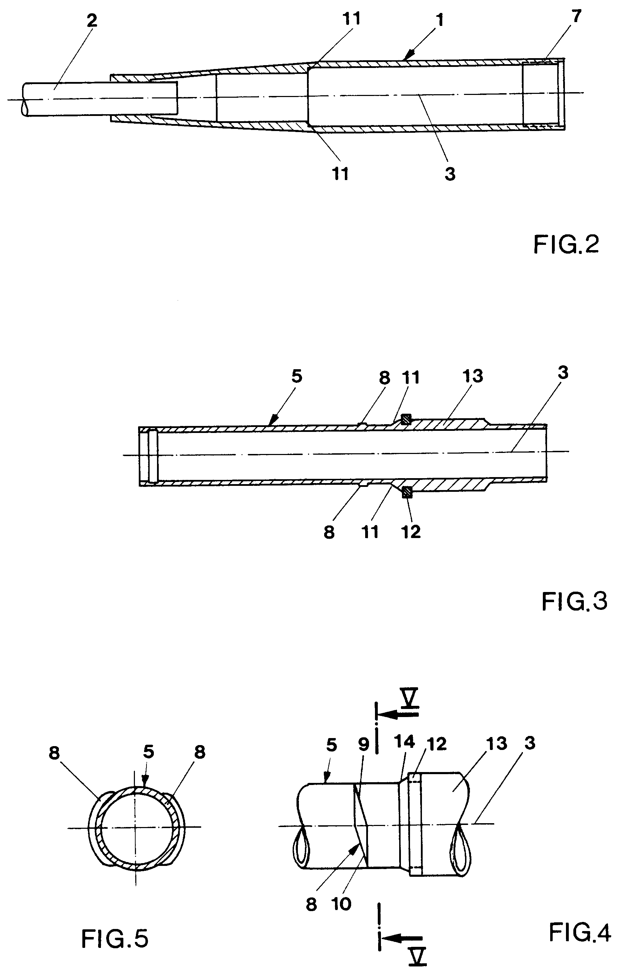

Wie in Figur 2 angedeutet ist, sind die Vertiefungen oder Nuten 7 an dem Innenumfang des Griffstücks 1 an dessen Ende vorgesehen, und zwar in diesem Ausführungsbeispiel in Form von zwei parallel zueinander verlaufenden Gewindegängen mit zwei Einführstellen für die Stege 8 an gegenüberliegenden Stellen des hülsenförmigen Griffstücks 1. Die Stege 8, von denen in diesem Ausführungsbeispiel zwei vorgesehen sind, sind an den gegenüberliegenden Seiten der Verbindungshülse 5 angeordnet, wie insbesondere die Figur 5 zeigt. Die zwei Stege 8 laufen jeweils zu ihren beiden Enden hin spitz aus, wie dies in der vergrößerten Auschnittsdarstellung der Figur 4 zu sehen ist.

Jeweils zwei der gegenüberliegenden Seiten 9, 10 jedes Steges 8 verlaufen parallel zueinander, wobei die eine Seite 9 bei Eindrehen der Verbindungshülse 5 in das Griffstück 1 eine kraftschlüssige Anlagefläche bildet, während die andere Seite 10 des Steges beim Herausdrehen der Verbindungshülse 5 aus dem Griffstück 1 eine kraftschlüssige Anlagefläche bildet. Durch die spitz auslaufenden Enden der Stege 8 ist ein leichtes Einführen der Stege in die beiden Vertiefungen 7 des Griffstücks 1 gegeben, zumal die Vertiefungen 7 mit einer Breite von etwa 2 mm einen ausreichend großen Einführbereich für die spitz zulaufenden Stege 8 bilden.

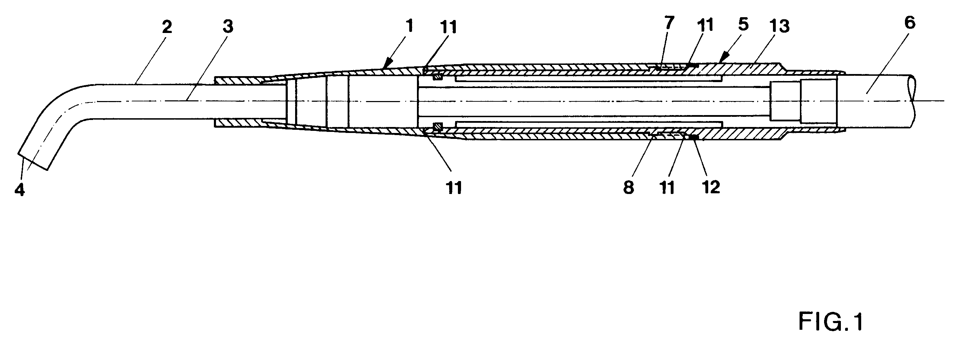

Die Steigung der Stege 8 bzw. der Führungsflächen 9 bzw. deren Neigungswinkel zu der Ebene, die senkrecht zu der Achse 3 verläuft, ist so gewählt, daß sich beim Eindrehen der Verbindungshülse 5 in das Griffstück 1 bei einer Verdrehung der beiden Teile gegeneinander um 360° eine Verschiebung in Richtung der Achse 3 von etwa 10 mm ergeben würde. Die Drehverbindung in diesem Ausführungsbeispiel ist derart ausgelegt, daß die beiden Teile mit einer halben Umdrehung in einer Stellung verriegelt sind, die in Figur 1 im Schnitt dargestellt ist, wobei entsprechende Anschlagflächen 11 am hinteren Ende der Verbindungshülse 5 und im vorderen Bereich an dem Innenumfang des Griffstücks 1 vorgesehen sind, um die beiden Teile im verschraubten Zustand zusätzlich in axialer Richtung gegeneinander festzulegen.

Im Bereich der Anschlagflächen 11 an der Außenseite der Verbindungshülse 5 ist ein Zwischenring 12 eingefügt, der sowohl in Figur 1 als auch in Figur 4 zu sehen ist, wobei darüberhinaus der Übergangsbereich zwischen dem Durchmesser der Verbindungshülse 5, der auf der Außenseite die beiden Stege 8 trägt, und dem größeren Außendurchmesser des Endabschnitts 13 der Verbindungshülse 5 ein schräger Übergangsbereich 14 ausgebildet ist, der eine zusätzliche Zentrierung des Endes des Griffstücks 1 an der Verbindungshülse 5 bildet.

Durch die relativ große Steigung der Stege 8 und der beiden auf einer gleichen Umfangslinie liegenden Stege 8, die jeweils in einer eigenen Vertiefung oder Nut 7 in den Innenumfang des Griffstücks 1 eintreten, reicht es bereis aus, die beiden Teile durch eine Verdrehung gegeneinander um eine halbe Umdrehung zu verriegeln bzw. wieder aus ihrem Eingriff miteinander zu lösen.

Die dargestellte Lichtzuführeinrichtung ist insbesondere für zahntechnische Anwendungen geeignet, beispielsweise zur Aushärtung von lichtpolymerisierenden Kunststoffen im Mund eines Patienten.

## Patentansprüche

1. Lichtzuführeinrichtung für medizinische und/oder zahntechnische Anwendungen mit einem Lichtleiter und einem Lichtaustrittsteil an deren einem Ende, das in einem Griffstück gehalten ist, wobei das Griffstück lösbar mittels Rastelementen mit einer Verbindungshülse verbunden ist, die ihrerseits unverrückbar an dem Lichtleiter befestigt ist, wobei die Rastelemente durch zwei Vorsprünge und diesen zugeordneten Vertiefungen gebildet sind, dadurch gekennzeichnet, daß die Vorsprünge und die Vertiefungen Teile einer Drehverbindung sind, wobei die Vorsprünge als Stege (8) ausgebildet sind.

2. Lichtleiterzuführeinrichtung nach Anspruch 1, dadurch gekennzeichnet, daß zwei voneinander getrennte Stege (8) vorgesehen sind.

3. Lichtleiterzuführeinrichtung nach Anspruch 1 oder 2, dadurch gekennzeichnet, daß die Stege (8) in Eindrehrichtung spitz zulaufen.

4. Lichtleiterzuführeinrichtung nach einem der Ansprüche 1 bis 3, dadurch gekennzeichnet, daß die Stege (8) an der Verbindungshülse (5) angeordnet sind und sich über einen Teil des Außenumfangs der Verbindungshülse (5) erstrecken.

5. Lichtleiterzuführeinrichtung nach einem der Ansprüche 1 bis 4, dadurch gekennzeichnet, daß die Stege (8) an beiden Enden spitz zulaufen.

6. Lichtleiterzuführeinrichtung nach einem der Ansprüche 1 oder 5, dadurch gekennzeichnet, daß die zwei Stege (8) in Umfangsrichtung der Verbindungshülse bzw. des Griffstücks (1) gesehen, radial gegenüberliegend verteilt sind.

7. Lichtleiterzuführeinrichtung nach einem der Ansprüche 1 bis 6, dadurch gekennzeichnet, daß die Vertiefungen (7) in Form von zwei parallel zueinander verlaufenden Gewindegängen am Innenumfang des Griffstücks (1) gebildet sind.

8. Lichtleiterzuführeinrichtung nach einem der Ansprüche 1 bis 7, dadurch gekennzeichnet, daß die Drehverbindung Gewindegänge aufweist, die eine Steigung von 5 bis 10 mm bei einer vollständigen Verdrehung der Verbindungshülse (5) relativ zu dem Griffstück (1) besitzen.

9. Lichtleiterzuführeinrichtung nach Anspruch 8, dadurch gekennzeichnet, daß die Steigung etwa 10 mm bei einer vollständigen Umdrehung beträgt.

10. Lichtleiterzuführeinrichtung nach einem der Ansprüche 1 bis 9, dadurch gekennzeichnet, daß die Stege (8) eine Breite vom 1 bis 3 mm aufweisen.

11. Lichtleiterzuführeinrichtung nach Anspruch 10, dadurch gekennzeichnet, daß die Breite der Stege (8) etwa 2 mm beträgt.

12. Lichtleiterzuführeinrichtung nach einem der Ansprüche 1 bis 11, dadurch gekennzeichnet, daß das Griffstück (1) aus sterilisierbarem Material besteht.

13. Lichtleiterzuführeinrichtung nach einem der Ansprüche 1 bis 12, dadurch gekennzeichnet, daß das Griffstück (1) und/oder die Verbindungshülse (5) aus Polyetherimid bestehen.
